# FASCICULE DE BREVET EUROPEEN

(11) **EP 3 355 832 B1**
(45) Date de publication et mention de la délivrance du brevet: **05.02.2020**
(21) Numéro de dépôt: 16774500.9
(22) Date de dépôt: 29.09.2016
(51) Int. Cl.: A61F 2/34, A61F 2/46, A61F 2/30

(54) **COTYLE D'UNE PROTHÈSE DE HANCHE ET PROTHÈSE DE HANCHE LE COMPORTANT**
PFANNE FÜR EINE HÜFTPROTHESE UND HÜFTPROTHESE DAMIT
ACETABULUM FOR A HIP PROSTHESIS AND HIP PROSTHESIS COMPRISING SAME

(30) Priorité: 30.09.2015 FR 1559285
(43) Date de publication de la demande: 08.08.2018
(73) Titulaire: Porte, Michel, 20166 Porticcio (FR); Doursounian, Levon, 75116 Paris (FR)
(72) Inventeur: Porte, Michel, 20166 Porticcio (FR); Doursounian, Levon, 75116 Paris (FR)
(74) Mandataire: Lavoix
(86) Numéro de dépôt international: PCT/EP2016/073261
(87) Numéro de publication internationale: WO 2017/055452

(56) Documents cités:
- WO-A1-2010/081996
- DE-A1-102008 047 627
- FR-A1- 2 789 570
- FR-A1- 2 903 881
- US-B1- 8 663 336

## Description

L'invention concerne le domaine des prothèses de hanche, et plus particulièrement les prothèses nécessitant la mise en place d'un dispositif destiné à reconstituer une cavité cotyloïdienne en vue d'une réception fonctionnellement satisfaisante de la tête d'une prothèse fémorale.

Pour reconstituer une cavité cotyloïdienne il est habituel de mettre en place un cotyle prothétique fixé par un ciment, ou par impaction, ou par des vis, dans le cotyle naturel, préalablement conformé à l'aide d'un outil adéquat (« fraise à cotyle ») pour la réception de ce cotyle prothétique. Cependant, l'orientation exacte du cotyle prothétique à implanter est très délicate, dans la mesure où les repères anatomiques ne sont pas fiables. En fonction de l'anatomie exacte du patient, une même orientation peut s'avérer adéquate ou, au contraire, inadaptée car susceptible de conduire trop aisément à des luxations de l'articulation.

On a déjà proposé, dans le passé, des cotyles dits « cotyles d'essai », dont l'orientation, après leur implantation dans la cavité cotyloïdienne naturelle, peut être réglée par le chirurgien. De cette façon, après avoir testé la pertinence de cette orientation avec les pièces fémorales d'essai afin de voir si elle ne conduit pas à un risque de luxation excessif, le chirurgien a la possibilité de rectifier cette orientation jusqu'à l'obtention d'un résultat satisfaisant du point de vue de la mobilité de l'articulation, puis de substituer à ce cotyle d'essai le cotyle définitif en lui conférant l'orientation choisie.

En particulier, le document EP-B-1 105 071 propose un tel cotyle d'essai comportant :
- une cupule hémisphérique à implanter dans la cavité cotyloïdienne ;
- un insert hémisphérique destiné à être engagé de façon concentrique dans ladite cupule ;
- une embase extérieure en forme de calotte sphérique, pourvue de plots d'ancrage dans la cavité cotyloïdienne de l'os iliaque et destinée à recevoir la cupule ;
- une coupelle intérieure destinée à être placée dans la cupule en regard de l'embase extérieure ;
- des moyens de liaison de l'embase et de la coupelle, ces deux pièces définissant des moyens de réglage de la position angulaire de la cupule dans la cavité cotyloïdienne en fonction de l'orientation à donner à l'articulation ;
- et des moyens d'immobilisation de la cupule par rapport à l'embase après le réglage de ladite position angulaire de la cupule.

Cette solution présente cependant des inconvénients. En premier lieu, les possibilités d'orientation de la cupule sont relativement limitées par le fait que l'ancrage de l'embase ne peut plus être modifié après son implantation dans la cavité cotyloïdienne. S'il s'avère, lors des essais, que la position de l'embase ne permet pas l'obtention d'une orientation satisfaisante de l'articulation, cette position ne peut être corrigée. De plus, la préhension des différentes pièces est difficile et nécessite l'utilisation d'ancillaires complexes. Enfin, le nombre de pièces mises en jeu rend ce type de prothèse coûteux à fabriquer.

Un autre type de cotyle d'essai n'ayant pas les inconvénients précités a été proposé selon le document WO-A-2010/081996.

Ledit cotyle comprend une cupule de forme générale hémisphérique destinée à être mise en place dans une cavité cotyloïdienne d'un os, un insert destiné à être placé à l'intérieur de ladite cupule et des moyens d'ancrage par percussion (tels que des agrafes) du cotyle dans la surface de la cavité cotyloïdienne. Ledit insert est une bague placée dans un logement de la cupule et définissant, en coopération avec la cupule, une cavité sphérique pour la réception de la tête sphérique de l'autre élément de la prothèse. Ladite bague intègre lesdits moyens d'ancrage par percussion, ceux-ci pouvant traverser la cupule de manière à ce que, lors de l'implantation du cotyle, la cupule se retrouve coincée entre ladite bague et la surface de ladite cavité cotyloïdienne. Ladite bague comporte des moyens assurant sa préhension par un ancillaire pour l'application d'un effort de traction permettant de supprimer ledit ancrage de la bague dans l'os.

Ce dernier modèle procure au chirurgien une très large faculté d'orientation du cotyle de manière à conformer la prothèse idéalement en fonction de l'anatomie exacte du patient, en vue d'éviter les luxations de l'articulation après l'implantation de la prothèse définitive.

Cependant les deux types de cotyle d'essai décrits précédemment nécessitent de luxer l'articulation coxo-fémorale du patient pour pouvoir modifier l'orientation du cotyle. Dans les deux cas le système d'ancrage osseux se retire par une traction axiale exercée par un instrument ancillaire placé sur la face équatoriale du cotyle d'essai et dans son axe. Il faut donc que la tête fémorale prothétique ne soit pas dans la cupule d'essai pour pouvoir modifier l'orientation de la cupule. Autrement dit, il faut que le chirurgien luxe la hanche du patient avant de modifier l'orientation du cotyle d'essai, et ensuite réduise l'articulation afin de refaire des essais. On conçoit bien que cette façon de procéder nécessite des manipulations supplémentaires toujours contraignantes pour le chirurgien et qu'elles allongent la durée de l'intervention, avec tous les inconvénients que cela implique, en particulier pour le patient, pour qui les multiples luxations qui peuvent être nécessaires présentent des risques de lésions sérieuses.

FR-A-2789579 divulgue une prothèse cotyloïdienne ayant une gorge périphérique pour recevoir une ceinture rétractable et munie des dents de fixation. Les extrémités de la ceinture sont rapprochées provisoirement par une broche pour rétracter la ceinture.

DE-A- 102008047627 divulgue un cotyle dont un élément élastique exerce un effort sur la cavité cotyloïdienne de manière à permettre une fixation libérable du cotyle.

Le but de l'invention est de procurer aux chirurgiens orthopédistes un cotyle ne nécessitant pas la luxation de la hanche du patient pour le réglage de son orientation.

A cet effet, l'invention a pour objet un cotyle orientable pour prothèse de hanche, comportant une cupule de forme générale hémisphérique destinée à être placée dans la cavité cotyloïdienne d'un patient, et un dispositif de maintien de l'orientation angulaire de ladite cupule dans ladite cavité cotyloïdienne, ledit dispositif de maintien comportant un anneau élastique présentant une fente transversale délimitée par deux bords de l'anneau, l'anneau étant de forme générale circulaire, et sa périphérie externe comportant des aspérités pouvant s'enfoncer dans la surface de la cavité cotyloïdienne lorsque l'anneau élastique est à l'état détendu, ledit anneau élastique comportant des moyens de préhension par un ancillaire de serrage permettant de déformer l'anneau élastique, dans le sens d'une réduction de son diamètre, y compris après que le cotyle assemblé a été placé dans la cavité cotyloïdienne, par un resserrement tendant à rapprocher les bords de l'anneau délimitant la fente, les formes et dimensions des différentes parties de l'anneau étant conçues pour que, dans l'état resserré de l'anneau, les aspérités ne dépassent pas de la surface externe de la cupule, rendant ainsi possible une modification de l'orientation angulaire de la cupule, et permettant que ladite modification puisse être effectuée sans qu'il soit nécessaire de luxer l'articulation coxo-fémorale du patient.

Selon une variante, la cupule a été réalisée en matériau polymère, par un surmoulage sur l'anneau élastique tout en ménageant des espaces autour de l'anneau élastique qui lui permettent de se déformer lors du réglage de l'orientation du cotyle.

Selon une autre variante, la cupule comporte une seule partie et est pourvue sur sa surface externe d'une rainure donnant accès à un espace à l'intérieur duquel l'anneau élastique peut se déformer en passant dudit état détendu audit état resserré et inversement.

Selon une autre variante, la cupule comporte au moins deux parties solidarisables l'une à l'autre, ledit anneau élastique étant pris en sandwich entre deux desdites parties, et lesdites deux parties définissent entre elles un espace à l'intérieur duquel l'anneau élastique peut se déformer en passant dudit état détendu audit état resserré et inversement.

Lesdits moyens de préhension de l'anneau élastique peuvent être constitués par des blocs reliés à l'anneau ou venus de matière avec lui, et pourvus de moyens pour la mise en prise d'un ancillaire de serrage, lesdits moyens étant accessibles par le chirurgien sans nécessité de luxer l'articulation coxo-fémorale du patient.

Ledit anneau élastique peut présenter plusieurs rangées d'aspéritéss disposées à des hauteurs différentes sur son épaisseur.

Il peut s'agir d'un cotyle d'essai.

Il peut s'agir d'un cotyle définitif.

L'invention a également pour objet une prothèse de hanche d'essai ou définitive comportant un cotyle et un élément fémoral, caractérisé en ce que ledit cotyle est un cotyle du type précédent.

Comme on l'aura compris, l'invention consiste à intégrer au cotyle un anneau élastique de forme générale circulaire, présentant une fente transversale, et dont la périphérie externe comporte des aspérités, telles que des dents, destinées à s'enfoncer dans la surface de la cavité cotyloïdienne de façon à y assurer l'ancrage de la cupule selon l'angulation choisie par le chirurgien. L'anneau est également pourvu de moyens de préhension par un ancillaire tel qu'une pince, qui permettent au chirurgien de déformer l'anneau, dans le sens d'une réduction de son diamètre, par un resserrement tendant à rapprocher les bords de l'anneau délimitant la fente. Les dimensions des différentes parties de l'anneau (diamètre extérieur, taille des aspérités, largeur de la fente...) et de la cupule sont conçues pour que, dans l'état resserré de l'anneau, les aspérités ne dépassent plus sensiblement de la surface externe de la cupule. De cette façon, avec l'anneau à l'état resserré, la cupule peut tourner librement dans la cavité cotyloïdienne de façon à permettre au chirurgien de modifier son orientation. Lorsque le chirurgien a réglé cette orientation, il desserre l'anneau en agissant sur l'ancillaire, et l'anneau reprend sa forme nominale avec les aspérités qui dépassent de la cupule et vont s'ancrer dans la surface de la cavité cotyloïdienne.

Une caractéristique remarquable de l'invention est que les moyens de préhension de l'anneau sont conformés et dimensionnés pour qu'ils soient accessibles au chirurgien, par l'intermédiaire de l'ancillaire, sans qu'il soit nécessaire de luxer l'articulation coxo-fémorale du patient, avec les risques d'incidents que cela impliquerait, surtout si le réglage s'avère difficile et que plusieurs orientations successives doivent être testées.

Optimalement, la manipulation du cotyle avec l'anneau à l'état resserré est effectuée également au moyen de l'ancillaire de serrage, qui peut être maintenu sur l'anneau pendant toute la durée de l'intervention.

L'invention sera mieux comprise à la lecture de la description qui suit, donnée en référence aux figures annexées suivantes :

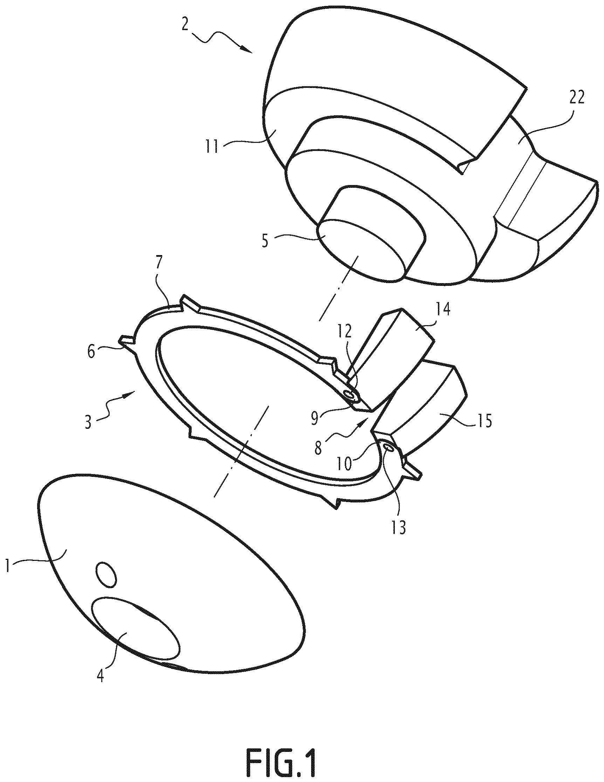

- La figure 1 qui montre en vue éclatée en perspective les différentes parties d'un exemple de cotyle selon l'invention ;
- La figure 2 qui montre ce même exemple de cotyle à l'état assemblé, vu de dessus, avec l'anneau élastique dans sa position resserrée ;
- La figure 3 qui montre ce même exemple de cotyle à l'état assemblé, vu de dessous, avec l'anneau élastique dans sa position resserrée, avec les aspérités (ici des dents) escamotées à l'intérieur du cotyle ;
- La figure 4 qui montre ce même exemple de cotyle à l'état assemblé, vu de dessous, avec l'anneau élastique dans sa position desserrée, avec les aspérités saillant hors de la surface extérieure du cotyle.

L'exemple de cotyle selon l'invention qui va être à présent décrit, et est représenté sur les figures, est un exemple de cotyle d'essai, destiné à être ensuite remplacé par un cotyle prothétique définitif qui sera orienté selon la direction que le cotyle d'essai selon l'invention aura permis de déterminer comme étant bien adaptée à la morphologie du patient. On verra plus loin que ce cas n'est, cependant, pas le seul auquel l'invention peut s'appliquer.

Le cotyle d'essai comporte une cupule en deux parties 1, 2 destinée à être insérées dans la cavité cotyloïdienne du patient, le cas échéant après une conformation de celle-ci par des méthodes classiques. A l'état assemblé, les deux parties 1, 2 forment une cupule de forme générale hémisphérique, comme cela est classique. La partie inférieure 1 est sensiblement en forme de calotte sphérique, et elle peut être fixée par tout moyen (vis, tenons...) sur la partie supérieure 2, les deux parties 1, 2 prenant alors en sandwich l'anneau élastique fendu 3 qui est un élément essentiel de l'invention. Un orifice 4 est ménagé au sommet de la calotte 1, pour y faire pénétrer une excroissance 5 de la partie supérieure 2, de façon à assurer un bon positionnement relatif des deux parties 1, 2. Le matériau de ces deux parties peut être, au choix, un métal, de préférence biocompatible (acier inoxydable, titane...), ou un polymère (polyéthylène, PEEK...). De manière générale, tout matériau (ou toute combinaison de matériaux) classiquement utilisable pour réaliser des cotyles de prothèse de hanche est envisageable.

Selon l'invention, l'anneau élastique fendu 3 comporte des moyens d'ancrage sous forme d'aspérités, telles que des dents 6 (qui sont au nombre de six dans l'exemple non limitatif représenté) disposées sur son bord extérieur 7. Ces dents (ou toutes aspérités permettant d'assurer l'ancrage de l'anneau) sont destinées à pénétrer dans la surface de la cavité cotyloïdienne du patient lorsque l'anneau élastique fendu 3 est à l'état détendu pour y ancrer le cotyle d'essai, et leurs dimensions et formes sont déterminées en conséquence, en relation avec les dimensions des autres éléments du cotyle selon l'invention, comme cela apparaîtra dans la suite de la description. Il comporte également une fente transversale 8, c'est-à-dire un espace qui interrompt sa périphérie 7 et permet une déformation allant dans le sens d'une diminution du diamètre de l'anneau élastique 3.

Les parties 1, 2 de la cupule du cotyle sont conformées pour, lorsqu'elles sont assemblées l'une sur l'autre, définir un logement à l'intérieur duquel l'anneau 3 peut être placé avec la possibilité de s'y contracter dans le sens d'une diminution de son diamètre, lorsqu'un effort est exercé sur lui par le chirurgien de part et d'autre de la fente 8 dans le sens d'un rapprochement des deux bords 9, 10 de l'anneau 3 délimitant la fente 8, puis de revenir à sa forme initiale lorsque l'effort cesse. Autrement dit, le dimensionnement du logement permet à l'anneau élastique 3 de passer de l'état détendu à l'état resserré et réciproquement. Ce logement peut être défini, comme représenté, par un palier 11 ménagé sur la périphérie de la partie supérieure 2 face à la partie inférieure 1.

Le matériau de l'anneau élastique 3 peut être choisi parmi les matériaux classiquement connus pour constituer des pièces élastiques résistantes, de préférence biocompatibles surtout dans le cas, envisageable, où il est appelé à subsister dans le corps du patient après la mise place de la prothèse. A cet effet on peut citer des aciers inoxydables à ressorts, par exemple de type X20Cr13, le titane et ses alliages, voire des polymères très résistants comme le PEEK (Polyétheréthercétone). L'essentiel est que la combinaison entre les dimensions de l'anneau élastique 3 et les caractéristiques mécaniques de son matériau permettent aux dents 6 d'exercer, lorsque l'anneau est détendu, un effort suffisant sur la surface de la cavité cotyloïdienne pour garantir leur pénétration dans cette surface et, ainsi, l'ancrage du cotyle selon l'angulation choisie par le chirurgien.

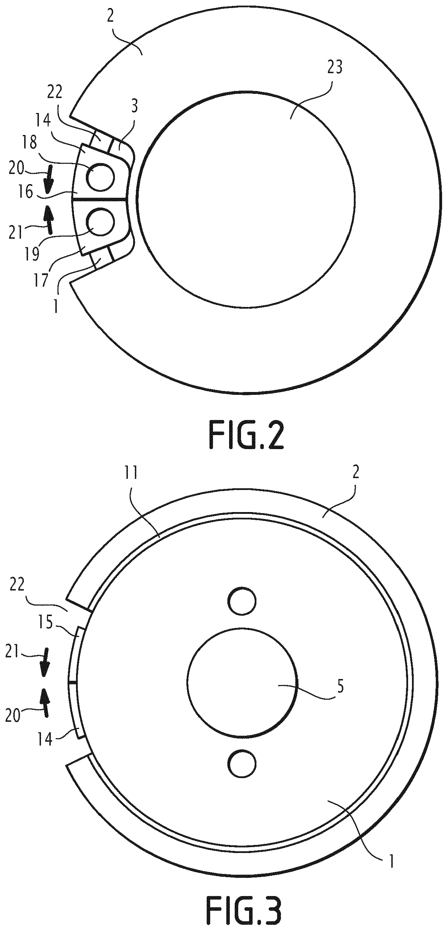

On peut imaginer diverses configurations pour permettre au chirurgien de réaliser la diminution du diamètre de l'anneau élastique fendu 3, une fois que le cotyle assemblé a été placé dans la cavité cotyloïdienne. Dans l'exemple représenté, l'anneau élastique fendu 3 comporte, au voisinage des bords 9, 10 délimitant la fente 8, des orifices 12, 13 dans lesquels on insère des tenons ménagés sur les surfaces inférieures respectives de deux blocs verticaux 14, 15. Ces blocs 14, 15 comportent chacun, sur leurs surfaces supérieures 16, 17 respectives, des logements 18, 19 dans lesquels le chirurgien peut insérer des tenons situés à l'extrémité d'un ancillaire de serrage (non représenté), qui est par exemple en forme de pince. En exerçant, au moyen de l'ancillaire, un effort selon les flèches 20, 21 tendant à rapprocher les blocs 14, 15 et à diminuer le diamètre général de l'anneau 3, le chirurgien parvient à la configuration représentée sur les figures 2 et 3, respectivement en vue de dessus et en vue de dessous. Les blocs 14, 15 peuvent se déplacer l'un vers l'autre du fait de la présence dans la partie supérieure 2 du cotyle d'une échancrure 22 à l'intérieur de laquelle ils peuvent se déplacer, de préférence, comme représenté, sans déborder de la surface externe du cotyle assemblé pour ne pas procurer au cotyle un encombrement qui pourrait être excessif et modifier le caractère sensiblement hémisphérique de la surface externe de la cupule.

Puis, tout en maintenant l'anneau 3 en position resserrée grâce, par exemple, à des moyens de blocage de l'écartement des tenons de l'ancillaire, le chirurgien peut modifier l'orientation du cotyle dans la cavité, par exemple en manipulant l'ancillaire. Une caractéristique remarquable de l'invention est que cette modification de l'orientation du cotyle peut s'effectuer sans qu'il soit nécessaire d'extraire la tête fémorale du logement 23 ménagé sur la face supérieure de la partie supérieure 2 du cotyle pour la réception de la tête du fémur ou la tête de l'élément fémoral de la prothèse, autrement dit de luxer l'articulation coxo-fémorale du patient, avec tous les risques et inconvénients dont on a parlé. Il faut simplement que l'ancillaire soit dimensionné pour que ses mouvements ne soient pas gênés par les tissus environnants, et que les logements 18, 19 ménagés sur les blocs 14, 15 (ou tout autre moyen de mise en prise de l'ancillaire sur les blocs 14, 15) soient accessibles au chirurgien sans déplacement de l'élément fémoral (d'essai ou définitif) de la prothèse. A cet effet, le placement des blocs 14, 15 (et, de façon générale, des moyens de préhension de l'anneau 3) de part et d'autre de la fente 8 et à proximité de ses bords est avantageux, en ce qu'il fait que le chirurgien n'a à exercer une action que sur un secteur angulaire unique et étroit de l'anneau 3.

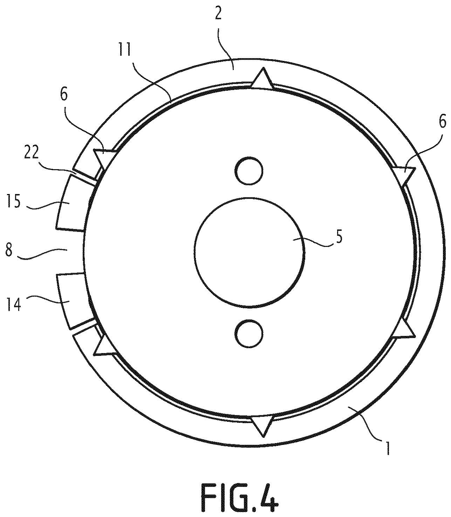

Lorsque le chirurgien pense avoir trouvé une orientation possiblement adéquate du cotyle dans la cavité cotyloïdienne, il fait cesser l'effort de serrage exercé sur l'anneau 3. Alors, le cotyle se retrouve dans l'état représenté sur la figure 4, dans lequel l'anneau élastique 3 a repris sensiblement sa forme naturelle, avec les dents 6 débordant de 1 ou plusieurs mm par rapport à la surface externe du cotyle, et pouvant donc s'enfoncer dans la surface de la cavité cotyloïdienne pour y ancrer le cotyle avec l'angulation choisie. Puis le chirurgien réalise les essais et mesures qui lui permettent de vérifier si l'orientation choisie est effectivement adéquate. Dans le cas contraire, il resserre l'anneau élastique 3 au moyen de l'ancillaire (qui, optimalement, est resté en place pendant toute la procédure) pour dégager les dents 6 de la paroi de la cavité cotyloïdienne, et il réoriente le cotyle jusqu'à une nouvelle position d'essai.

Lorsqu'une orientation adéquate du cotyle assemblé est clairement trouvée, deux cas peuvent se présenter.

Dans le premier cas, le cotyle n'est considéré que comme un cotyle d'essai. Alors, le chirurgien luxe l'articulation et remplace le cotyle selon l'invention par le cotyle définitif classique de son choix, auquel il confère l'orientation que le cotyle d'essai selon l'invention a permis de déterminer en en un minimum de temps et en écartant autant que possible les risques d'incidents liés à de multiples luxations. En effet, l'installation de la prothèse dans son orientation optimale ne nécessite au total que deux luxations, à savoir celle qui permet de dégager la cavité cotyloïdienne, de la conformer et d'y insérer le cotyle d'essai selon l'invention, et celle qui permet d'ôter le cotyle d'essai et de le remplacer par le cotyle définitif classique. Ce cotyle d'essai peut être à usage unique ou multiple, ce qui va gouverner notamment le choix de ses matériaux, par rapport à leurs solidités et leurs aptitudes ou non à être stérilisés de multiples fois.

Dans le deuxième cas, le cotyle selon l'invention peut constituer lui-même le cotyle définitif, en particulier si tous ses matériaux sont biocompatibles sur le long terme. L'installation et le réglage de la prothèse ne nécessitent donc plus que la première des deux luxations précitées, et les avantages de l'invention s'en trouvent encore augmentés. Pour cela, il faut :
- soit que la pénétration des dents 6, les efforts qu'elles exercent sur la paroi de la cavité et la force de maintien en position de l'anneau élastique 3 à l'intérieur du cotyle assemblé soient suffisants pour éviter un déplacement du cotyle par rapport à l'orientation finale, lors des activités ultérieures du patient ;
- soit que ce maintien en position définitive du cotyle soit assisté par d'autres moyens de fixation que l'anneau élastique 3 et ses dents 6, tels qu'un matériau de scellage ou des vis.

A titre d'exemple non limitatif, un cotyle d'essai selon l'invention tel qu'il vient d'être décrit peut présenter les caractéristiques et principales dimensions suivantes : diamètre extérieur de la partie supérieure 2 : 50 mm ; diamètre du logement 23 pour la tête fémorale : 28 mm ; matériau de l'anneau élastique 3 : acier à ressorts inoxydable X20Cr13 ; épaisseur de l'anneau élastique 3 : 1 mm ; diamètre intérieur de l'anneau élastique 3 : 36 mm ; diamètre extérieur de l'anneau élastique 3 : 43 mm ; longueur des dents 6 : 2,50 mm ; angle du sommet des dents 6 ; largeur de la fente 8 : 8,6 mm.

Il va de soi que ces caractéristiques dimensionnelles, qui ne sont qu'un exemple, doivent être adaptées (de façon, par exemple, homothétique à ce qui vient d'être dit) en fonction des mensurations précises du patient. Elles devraient être adaptées aussi si le cotyle était un cotyle définitif : en particulier l'anneau devrait généralement être plus épais, à savoir quelques mm.

Avantageusement, l'anneau 3 est disposé à un niveau du cotyle suffisamment bas pour que, quelle que soit l'orientation du cotyle au moins dans les cas les plus courants, l'intégralité de la périphérie de l'anneau soit située à l'intérieur de la cavité cotyloïdienne. Cette configuration est nettement plus avantageuse que si les moyens d'ancrage du cotyle étaient situés à sa partie supérieure ou à proximité immédiate de celle-ci, car elle garantit que l'ancrage du cotyle sera d'une qualité égale quelle que soit son orientation.

Bien entendu, des variantes de l'invention peuvent être imaginées. On va en citer quelques-unes, de façon non limitative.

Les blocs 14, 15, au lieu d'être des pièces séparées rapportées sur l'anneau 3 et pouvant éventuellement en être détachées après usage, peuvent être venus de matière avec lui.

Inversement, ces blocs 14, 15 peuvent être absents, et l'ancillaire peut venir en prise directement sur l'anneau élastique 3. La présence des blocs 14, 15 a cependant pour avantage de rapprocher les orifices 18, 19 de mise en prise de l'ancillaire par rapport à la surface supérieure du cotyle, et de les rendre ainsi bien visibles et accessibles par le chirurgien. De plus, comme représenté, il est possible de décaler sensiblement ces orifices 18, 19 de mise en en prise de l'ancillaire par rapport au cercle médian de l'anneau élastique 3 en direction de la périphérie extérieure de l'anneau, voire au-delà. Outre une facilitation de l'accès auxdits orifices, cela présente l'avantage d'augmenter le moment de déformation appliqué à l'anneau élastique 3, à effort exercé par l'ancillaire égal, par rapport au cas où les orifices de mise en prise se situeraient sur le cercle médian de l'anneau 3. Egalement, comme on le voit sur les figures 2, 3 et 4, la position plus ou moins rétractée des blocs 14, 15 est un indicateur visuel pour le chirurgien du degré de la rétractation des dents 6 ou de leur pénétration dans la paroi de la cavité cotyloïdienne.

L'anneau élastique 3 peut, éventuellement, présenter une épaisseur plus importante que ce qui est représenté, de manière à pouvoir être pourvu de plusieurs rangées de dents 6 (ou d'autres formes d'aspérités) situées à des hauteurs différentes sur son épaisseur, de façon à renforcer l'ancrage du cotyle selon l'invention et la stabilité de son orientation. Ce peut être particulièrement intéressant lorsque le cotyle est appelé à rester en place de façon définitive après que son orientation optimale a été déterminée.

La surface extérieure de la cupule du cotyle peut elle-même présenter des caractéristiques (filetages, rugosités...) qui, de façon connue, contribuent à son maintien à l'intérieur de la cavité cotyloïdienne dans la position visée par le chirurgien, que ce soit pendant la phase de recherche de l'orientation optimale ou surtout pendant la phase d'utilisation du cotyle si celui-ci est appelé à être définitif.

Le nombre de pièces constituant le cotyle peut être différent de ce qui a été décrit et représenté jusqu'ici. En plus de l'anneau élastique 3, il peut y avoir plus de deux autres pièces constituant la cupule, ou, au contraire, il peut n'y avoir qu'une seule autre pièce, auquel cas :
- Ladite pièce unique peut comporter une rainure sur sa surface externe, qui donne accès à un logement comparable à celui précédemment décrit et représenté ; l'insertion de l'anneau élastique 3 dans ladite rainure se fait alors par une augmentation immédiate ou progressive de son diamètre, réalisée par exemple en faisant glisser l'anneau 3 le long de la paroi externe du cotyle en partant de sa partie inférieure ;
- Ou le cotyle est réalisé par surmoulage d'un polymère sur l'anneau 3, les différents espaces permettant à l'anneau 3 de se déformer pendant le réglage de l'orientation du cotyle pouvant être ménagés, par exemple, au moyens d'entretoises que l'on détruit ensuite par un traitement thermique ou une attaque chimique.

L'invention concerne aussi une prothèse complète de hanche, d'essai ou définitive, c'est-à-dire un ensemble comportant, outre le cotyle qui vient d'être décrit et qui est destiné à être utilisé comme cotyle d'essai ou définitif, un élément fémoral d'essai ou définitif dont la tête sphérique est destinée à venir s'insérer dans le logement 23 de la surface supérieure de la cupule du cotyle. Cet élément fémoral n'a pas de raison particulière d'être différent d'un élément fémoral de prothèse de hanche plus classique, et il n'y a pas de nécessité à modifier les éléments fémoraux habituellement utilisés, pour les rendre compatibles avec le cotyle orientable sans luxation qui est à la base de l'invention. C'est aussi un des avantages de l'invention.

L'invention est applicable non seulement à la chirurgie orthopédique humaine, mais aussi à la chirurgie orthopédique vétérinaire (chiens, chats, chevaux...), avec des adaptations, notamment dimensionnelles, évidentes pour l'homme du métier, qui dépendent bien sûr de l'espèce animale concernée et de l'individu traité. Les problèmes à attendre pour l'adaptation de l'invention à la médecine vétérinaire ne dépassent pas, en nature et en difficulté, ceux qui sont habituellement rencontrés lors de la transposition d'une technique orthopédique connue d'un domaine dans un autre.

## Revendications

1. Cotyle orientable pour prothèse de hanche, comportant une cupule de forme générale hémisphérique destinée à être placée dans la cavité cotyloïdienne d'un patient, et un dispositif de maintien de l'orientation angulaire de ladite cupule dans ladite cavité cotyloïdienne, ledit dispositif de maintien comportant un anneau élastique (3) présentant une fente transversale (8) délimitée par deux bords (9, 10) de l'anneau (3), l'anneau (3) étant de forme générale circulaire, et sa périphérie externe comportant des aspérités (6) pouvant s'enfoncer dans la surface de la cavité cotyloïdienne lorsque l'anneau élastique (3) est à l'état détendu, **caractérisé en ce que** l'anneau élastique (3) comporte des moyens de préhension par un ancillaire de serrage permettant de déformer l'anneau élastique (3), dans le sens d'une réduction de son diamètre, y compris après que le cotyle assemblé a été placé dans la cavité cotyloïdienne, par un resserrement tendant à rapprocher les bords (9, 10) de l'anneau (3) délimitant la fente (8), les formes et dimensions des différentes parties de l'anneau (3) étant conçues pour que, dans l'état resserré de l'anneau (3), les aspérités (6) ne dépassent pas de la surface externe de la cupule, rendant ainsi possible une modification de l'orientation angulaire de la cupule, et permettant que ladite modification puisse être effectuée sans qu'il soit nécessaire de luxer l'articulation coxo-fémorale du patient.

2. Cotyle orientable selon la revendication 1, **caractérisé en ce que** la cupule a été réalisée en matériau polymère, par un surmoulage sur l'anneau élastique (3) tout en ménageant des espaces autour de l'anneau élastique (3) qui lui permettent de se déformer lors du réglage de l'orientation du cotyle.

3. Cotyle orientable selon la revendication 1, **caractérisé en ce que** la cupule comporte une seule partie et est pourvue sur sa surface externe d'une rainure donnant accès à un espace à l'intérieur duquel l'anneau élastique (3) peut se déformer en passant dudit état détendu audit état resserré et inversement.

4. Cotyle orientable selon la revendication 1, **caractérisé en ce que** la cupule comporte au moins deux parties (1, 2) solidarisables l'une à l'autre, ledit anneau élastique (3) étant pris en sandwich entre deux (1, 2) desdites parties, et **en ce que** lesdites deux parties (1, 2) définissent entre elles un espace à l'intérieur duquel l'anneau élastique (3) peut se déformer en passant dudit état détendu audit état resserré et inversement.

5. Cotyle orientable selon l'une des revendications 1 à 4, **caractérisé en ce que** lesdits moyens de préhension de l'anneau élastique sont constitués par des blocs (14, 15) reliés à l'anneau (3) ou venus de matière avec lui, et pourvus de moyens (18, 19) pour la mise en prise d'un ancillaire de serrage, lesdits moyens (18, 19) étant accessibles par le chirurgien sans nécessité de luxer l'articulation coxo-fémorale du patient.

6. Cotyle orientable selon l'une des revendications 1 à 5, **caractérisé en ce que** les moyens de préhension de l'anneau (3) sont situés de part et d'autre de la fente (8) et à proximité de ses bords.

7. Cotyle orientable selon l'une des revendications 1 à 6, **caractérisé en ce que** ledit anneau élastique (3) présente plusieurs rangées d'aspérités (6) disposées à des hauteurs différentes sur son épaisseur.

8. Cotyle orientable selon l'une des revendications 1 à 7, **caractérisé en ce qu'**il s'agit d'un cotyle d'essai.

9. Cotyle orientable selon l'une des revendications 1 à 7, **caractérisé en ce qu'**il s'agit d'un cotyle définitif.

10. Prothèse de hanche comportant un cotyle et un élément fémoral, **caractérisé en ce que** ledit cotyle est du type selon l'une des revendications 1 à 9.

## Patentansprüche

1. Ausrichtbare Gelenkpfanne für eine Hüftgelenkprothese, aufweisend einen Becher von im Allgemeinen halbkugelförmiger Form, der dazu bestimmt ist, in der Gelenkkavität eines Patienten platziert zu werden, und eine Vorrichtung zum Aufrechterhalten der Winkelausrichtung des Bechers in der Gelenkkavität, wobei die Vorrichtung zum Aufrechterhalten aufweist einen elastischen Ring (3), der einen Querschlitz (8) hat, der begrenzt ist von zwei Rändern (9, 10) des Rings (3), wobei der Ring (3) im Allgemeinen von kreisförmiger Form ist, wobei sein Außenumfang Unebenheiten (6) aufweist, die sich in die Fläche der Gelenkkavität eindrücken können, wenn der elastische Ring (3) im entspannten Zustand ist, **dadurch gekennzeichnet, dass** der elastische Ring (3) aufweist Mittel zum Ergriffen-Werden von einer Spannhilfe, es erlaubend, den elastischen Ring (3) zu deformieren im Sinne einer Verkleinerung seines Durchmessers, einschließlich nachdem das zusammengebaute Gelenk in der Gelenkkavität platziert worden ist, durch ein Verengen, das dazu tendiert, die Ränder (9, 10) des Rings (3), die den Schlitz (8) begrenzen, anzunähern, wobei die Formen und Abmessungen der unterschiedlichen Abschnitte des Rings (3) derart konzipiert sind, damit im verengten Zustand des Rings (3), die Unebenheiten (6) nicht über die Außenfläche des Bechers hinausstehen, wodurch eine Modifikation der Winkelausrichtung des Bechers ermöglicht wird und es ermöglicht ist, dass die Modifikation durchgeführt werden kann, ohne dass es erforderlich ist, das Hüftgelenk des Patienten auszukugeln.

2. Ausrichtbare Gelenkpfanne gemäß Anspruch 1, **dadurch gekennzeichnet, dass** die Gelenkpfanne realisiert ist aus einem Polymermaterial durch ein Auf-Formen auf den elastischen Ring (3) unter Aussparung der Räume um den elastischen Ring (3), die es ihm erlauben, sich während der Einstellung der Ausrichtung der Gelenkpfanne zu deformieren.

3. Ausrichtbare Gelenkpfanne gemäß Anspruch 1, **dadurch gekennzeichnet, dass** der Becher ein einziges Teil aufweist und an seiner Außenfläche mit einer Nut versehen ist, die Zugang zu einem Raum gibt, in dessen Inneres sich der elastische Ring (3) deformieren kann beim Übergehen von dem entspannten Zustand in den verengten Zustand und umgekehrt.

4. Ausrichtbare Gelenkpfanne gemäß Anspruch 1, **dadurch gekennzeichnet, dass** der Becher wenigstens zwei Teile (1, 2) aufweist, die fest miteinander verbindbar sind, wobei der elastische Ring (3) zwischen zweien (1, 2) dieser Teile Sandwich-artig angeordnet ist, und dass die beiden Teile (1, 2) zwischen sich einen Raum definieren, in dessen Inneres sich der elastische Ring (3) deformieren kann beim Übergehen von dem entspannten Zustand in den verengten Zustand und umgekehrt.

5. Ausrichtbare Gelenkpfanne gemäß einem der Ansprüche 1 bis 4, **dadurch gekennzeichnet, dass** die Mittel zum Ergriffen-Werden des elastischen Rings gebildet sind von Blöcken (14, 15), die mit dem Ring (3) verbunden sind oder aus einem Stück mit diesem sind, und welche versehen sind mit Mitteln (18, 19) zum In-Eingriff-Stehen mit einer Spannhilfe, wobei die Mittel (18, 19) zugänglich sind für den Chirurgen, ohne die Notwendigkeit, das Hüftgelenk des Patienten auszukugeln.

6. Ausrichtbare Gelenkpfanne gemäß einem der Ansprüche 1 bis 5, **dadurch gekennzeichnet, dass** die Mittel zum Ergriffen-Werden des Rings (3) beidseitig des Schlitzes (8) nahe seiner beiden Ränder angeordnet sind.

7. Ausrichtbare Gelenkpfanne gemäß einem der Ansprüche 1 bis 6, **dadurch gekennzeichnet, dass** der elastische Ring (3) mehrere Reihen von Unebenheiten (6) hat, die mit unterschiedlichen Höhen auf seiner Dicke angeordnet sind.

8. Ausrichtbare Gelenkpfanne gemäß einem der Ansprüche 1 bis 7, **dadurch gekennzeichnet, dass** es sich um eine Probe-Gelenkpfanne handelt.

9. Ausrichtbare Gelenkpfanne gemäß einem der Ansprüche 1 bis 7, **dadurch gekennzeichnet, dass** es sich um eine endgültige Gelenkpfanne handelt.

10. Hüftgelenkprothese mit einer Gelenkpfanne und einem Oberschenkelelement, **dadurch gekennzeichnet, dass** die Gelenkpfanne vom Typ gemäß einem der Ansprüche 1 bis 9 ist.

## Claims

1. An adjustable acetabulum for a hip prosthesis, including a generally hemispherical cup configured to be placed in the acetabular cavity of a patient, and a device for maintaining the angular orientation of said cup in said acetabular cavity, said maintaining device including a resilient ring (3) having a transverse slit (8) delimited by two edges (9, 10) of the ring (3), the ring (3) being generally circular, and its outer periphery including asperities (6) that can penetrate into the surface of the acetabular cavity when the resilient ring (3) is in the relaxed state, **characterized in that** the resilient ring (3) includes means for gripping by an ancillary gripping device making it possible to deform the resilient ring (3), in the direction of a reduction of its diameter, including after the assembled acetabulum has been placed in the acetabular cavity, by a tightening tending to bring the edges (9, 10) of the ring (3) delimiting the slit (8) closer together, the shapes and sizes of the different parts of the ring (3) being designed so that, in the tightened state of the ring (3), the asperities (6) do not protrude from the outer surface of the cup, thus allowing a modification of the angular orientation of the cup, and making it possible for said modification to be able to be done without it being necessary to dislocate the coxo-femoral joint of the patient.

2. The adjustable acetabulum according to claim 1, **characterized in that** the cup has been made from a polymeric material, by overmolding on the resilient ring (3) while arranging spaces around the resilient ring (3) that allow it to deform during the adjustment of the orientation of the acetabulum.

3. The adjustable acetabulum according to claim 1, **characterized in that** the cup includes a single part and is provided on its outer surface with a groove providing access to a space inside which the resilient ring (3) can deform by going from said relaxed state to said tightened state and vice versa.

4. The adjustable acetabulum according to claim 1, **characterized in that** the cup includes at least two parts (1, 2) able to be secured to one another, said resilient ring (3) being sandwiched between two (1, 2) of said parts, and **in that** said two parts (1, 2) define a space between them inside which the resilient ring (3) can deform by going from said relaxed state to said tightened state and vice versa.

5. The adjustable acetabulum according to one of claims 1 to 4, **characterized in that** said means for gripping the resilient ring are made up of blocks (14, 15) coupled to the ring (3) or integral therewith, and provided with means (18, 19) for engaging an ancillary gripping device, said means (18, 19) being accessible by the surgeon without having to dislocate the coxo-femoral joint of the patient.

6. The adjustable acetabulum according to one of claims 1 to 5, **characterized in that** the means for gripping the ring (3) are located on either side of the slit (8) and near its edges.

7. The adjustable acetabulum according to one of claims 1 to 6, **characterized in that** said resilient ring (3) has several rows of asperities (6) arranged at different heights over its thickness.

8. The adjustable acetabulum according to one of claims 1 to 7, **characterized in that** it is a trial acetabulum.

9. The adjustable acetabulum according to one of claims 1 to 7, **characterized in that** it is a permanent acetabulum.

10. A hip prosthesis including an acetabulum and a femoral element, **characterized in that** said acetabulum is of the type according to one of claims 1 to 9.
